# EUROPEAN PATENT APPLICATION

(11) **EP 1 627 654 A1**
(43) Date of publication of application: **22.02.2006**
(21) Application number: 04733158.2
(22) Date of filing: 14.05.2004
(51) Int. Cl.: A61M 15/00

(54) **INHALATION TYPE DOSING DEVICE**

(30) Priority: 28.05.2003 JP 2003150899
(71) Applicant: Hitachi Ltd., Tokyo 100-8280 (JP); DOTT LIMITED COMPANY, Yokohama-shi, Kanagawa 224-0051 (JP)
(72) Inventor: OHKI, Hisatomo c/o Automotive Systems, Isesaki-shi Gunma, 3720023 (JP); NAKAMURA, Shigemi c/o Automotive Systems, Isesaki-shi Gunma, 3720023 (JP); ISHIZEKI, Kazunori c/o Automotive systems, Isesaki-shi Gunma, 3720023 (JP); YANAGAWA, Akira, Yokohama-shi, Kanagawa 224-0051 (JP)
(74) Representative: Grünecker, Kinkeldey, Stockmair & Schwanhäusser Anwaltssozietät
(86) International application number: PCT/JP2004/006896
(87) International publication number: WO 2004/105844

(57) **Abstract**

Medicine powder is prevented from adhering to the tongue and the like when the medicine powder is being inhaled, discomfort due to the sense of taste is relieved, and a prescribed amount of medicine powder is enabled to be administered efficiently. With regard to a long mouthpiece 16, the length L of its held-in-the-mouth area 16C to be held in the mouth 19 is set at approximately 80mm. This enables one end of the held-in-the-mouth area 16C to be inserted deep into the oral cavity 22 when the held-in-the-mouth 16C is held in the mouth 19. Accordingly, the medicine powder can be discharged in a position beyond sweetness sensing parts 24A, saltiness sensing parts 24B, sourness sensing parts 24C and bitterness sensing parts 24D of the tongue 24. Thereby, a patient can inhale the medicine powder comfortably without sensing bitterness, sourness, sweetness and the like of the medicine powder. In addition, the held-in-the-mouth area 16C of the long mouthpiece 16 can press down the tongue 24, thus enabling the medicine powder to be prevented from adhering to the tongue and the like.

## Description

### FIELD OF THE INVENTION

The present invention relates to an inhalation-type medicine applicator which is preferable, for example, in being used for administering powdered medicine (medicine powder) to the lungs or the like of a patient through the patient's breath inhalation.

### BACKGROUND ART

In general, a method of inhaling powdered medicine (hereinafter referred to as "medicine powder") filled in a medicine powder container by use of a specialized medicine applicator has been known as a method of administering medicine to the lungs or the like of a patient suffering from asthma.

In addition, the inhalation-type medicine applicator includes a medicine powder container for containing medicine powder and a mouthpiece which is held in the mouth when the medicine powder in the medicine powder container is inhaled. The inside of the mouthpiece constitutes an admission port from which the medicine powder is discharged, and is open to the end of the mouthpiece. At this point, the length of the mouthpiece is set at a length which allows the end of the mouthpiece to barely pass over the front teeth when the mouthpiece is held in the mouth. In other words, the length of a part with which the mouthpiece is held in the mouth is set, for example, at approximately 15mm to 25mm (see Japanese Patent Laid-open Official Gazette No. Hei.7-313599, for example.).

Furthermore, in a case where medicine powder is going to be inhaled by use of an inhalation-type medicine applicator according to a conventional technology, the medicine powder container is filled with medicine powder, the mouthpiece is held in the mouth, and the breath is blown therein through the admission port. Thereby, the external air is flown into the medicine powder container, and this air flow supplies the medicine powder in the medicine powder container to the admission port, thus discharging the medicine powder through the admission port. By this, the medicine powder is administered to the lungs or the like.

With regard to the aforementioned inhalation-type medicine applicator according to the conventional technology, however, the medical power to be discharged from the admission port is liable to adhere to the tongue, since the length of the mouthpiece is set at a length (for example, 15mm to 25mm) which allows the end of the mouthpiece to barely pass over the front teeth when the mouthpiece is held in the mouth. This causes the patient to feel discomfort to bitterness, sourness, sweetness and the like of medicine powder when the medicine powder is administer. Accordingly, this brings about a problem that the medicine powder is hindered from being administered without discomfort.

Moreover, the medicine powder to be discharged from the admission port adheres to the tongue, the interior of the oral cavity and the like. This brings about another problem which hinders a prescribed amount of medicine powder filled in the medicine powder container from being administered to the lungs or the like of the patient.

### DISCLOSURE OF THE INVENTION

With the problems with the conventional technology taken into consideration, the present invention has been made. An object of the present invention is to prevent inhaled medicine powder from adhering to the tongue and the like, thereby relieving discomfort due to the taste sensation. Another object of the present invention is to provide an inhalation-type medicine applicator which enables a prescribed amount of medicine powder to be administered efficiently. In order to solve the aforementioned problems, the present invention according to claim 1 causes a mouthpiece, which is held in the mouth when medicine powder in the medicine powder container is inhaled, to have a configuration in which the length of its part with which the mouthpiece is held in the mouth is set at a range of 30mm to 80mm.

This configuration enables the end of the mouthpiece to be inserted deep into the oral cavity when the mouthpiece is held in the mouth. This enables the medicine powder to be discharged, for example, in a position beyond gustatory organs. Thereby, the medicine powder can be inhaled comfortably while taste of the medicine powder including bitterness, sourness, sweetness and the like is not being sensed when the medicine powder is inhaled.

In addition, the mouthpiece can press down the tongue which is an obstacle, thereby enabling the medicine powder to be discharged directly to the respiratory tract. This can prevent the medicine powder from adhering to the tongue, the internal surface of the oral cavity and the like. Accordingly, a prescribed amount of medicine powder can be administered to an affected part in the bronchi or the like efficiently.

With regard to the mouthpiece, it is preferable that the length of its part which is held in the mouth be set at a range of 40mm to 80mm in a case where medicine powder to be inhaled is medicine powder including sweetness. This enables the mouthpiece to discharge the medicine powder in a position beyond the front end of the tongue which senses sweetness. For this reason, the medicine powder can be inhaled comfortably while the sweetness of the medicine powder is not being sensed.

In addition, with regard to the mouthpiece, it is preferable that the length of its part which is held in the mouth be set at a range of 50mm to 80mm in a case where medicine powder to be inhaled is medicine powder including sourness. This enables the mouthpiece to discharge the medicine powder in a position beyond the side edges of the tongue which sense sourness. For this reason, the medicine powder can be inhaled comfortably while the sourness of the medicine powder is not being sensed.

Furthermore, with regard to the mouthpiece, it is preferable that the length of its part which is held in the mouth be set at a range of 60mm to 80mm in a case where medicine powder to be inhaled is medicine powder including bitterness. This enables the mouthpiece to discharge the medicine powder in a position beyond the deep part on the tongue which senses bitterness. For this reason, the medicine powder can be inhaled comfortably while the bitterness of the medicine powder is not being sensed. The length of the part of the mouthpiece which is held in the mouth is set at such a length that the admission port of the mouthpiece may be placed in the range from the vicinity of the gustatory organs which sense sweetness to the deep part of the oral cavity. This enables the medicine powder to be discharged in the position beyond the front end of the tongue which senses sweetness. For this reason, the medicine powder can be inhaled comfortably while the sweetness of the medicine powder is not being sensed.

Moreover, the length of the part of the mouthpiece which is held in the mouth is set at such a length that the admission port of the mouthpiece may be placed in the range from the vicinity of the gustatory organs which sense sourness to the deep part of the oral cavity. This enables the medicine powder to be discharged in the position beyond the side edges of the tongue which senses sourness. For this reason, the medicine powder can be inhaled comfortably while the sourness of the medicine powder is not being sensed.

Additionally, the length of the part of the mouthpiece which is held in the mouth is set at such a length that the admission port of the mouthpiece may be placed in the range from the vicinity of the gustatory organs which sense bitterness to the deep part of the oral cavity. This enables the medicine powder to be discharged in the position beyond the deep part of the tongue which senses bitterness. For this reason, the medicine powder can be inhaled comfortably while the bitterness of the medicine powder is not being sensed.

According to the present invention, the end of the mouthpiece can be inserted deep into the oral cavity when the mouthpiece is held in the mouth. This enables the medicine powder to be discharged, for example, in a position beyond the gustatory organs. Thereby, the medicine powder can be inhaled comfortably while taste of the medicine powder including bitterness, sourness, sweetness and the like is not being sensed when the medicine powder is inhaled. This mouthpiece can press down the tongue which is an obstacle, and thereby enables the medicine powder to be discharged directly to the respiratory tract. This can prevent the medicine powder from adhering to the tongue, the internal surface of the oral cavity and the like. Accordingly, a prescribed amount of medicine powder can be administered to an affected part in the bronchi or the like efficiently.

In addition, according to the present invention, the length of the part of the mouthpiece which is held in the mouth is set at the range of 40mm to 80mm in a case where medicine powder to be inhaled is medicine powder including sweetness. This enables the mouthpiece to discharge the medicine powder in the position beyond the front end of the tongue which senses sweetness. For this reason, the medicine powder can be inhaled comfortably while the sweetness of the medicine powder is not being sensed.

Furthermore, according to the present invention, the length of the part of the mouthpiece which is held in the mouth is set at the range of 50mm to 80mm in a case where medicine powder to be inhaled is medicine powder including sourness. This enables the mouthpiece to discharge the medicine powder in the position beyond the side edges of the tongue which sense sourness. For this reason, the medicine powder can be inhaled comfortably while the sourness of the medicine powder is not being sensed.

Moreover, according to the present invention, the length of the part of the mouthpiece which is held in the mouth is set at the range of 60mm to 80mm in a case where medicine powder to be inhaled is medicine powder including bitterness. This enables the mouthpiece to discharge the medicine powder in the position beyond the deep part of the tongue which senses bitterness . For this reason, the medicine powder can be inhaled comfortably while the bitterness of the medicine powder is not being sensed.

In addition, according to the present invention, the length of the part of the mouthpiece which is held in the mouth is set at such a length that the admission port of the mouthpiece may be placed in the range from the vicinity of the gustatory organs which sense sweetness to the deep part of the oral cavity. This enables the medicine powder to be discharged in the position beyond the front end of the tongue which senses sweetness. For this reason, the medicine powder can be inhaled comfortably while the sweetness of the medicine powder is not being sensed.

Furthermore, according to the present invention, the length of the part of the mouthpiece which is held in the mouth is set at such a length that the admission port of the mouthpiece may be placed in the range from the vicinity of the gustatory organs which sense sourness to the deep part of the oral cavity. This enables the medicine powder to be discharged in the position beyond the side edges of the tongue which sense sourness. For this reason, the medicine powder can be inhaled comfortably while the sourness of the medicine powder is not being sensed.

Moreover, according to the present invention, the length of the part of the mouthpiece which is held in the mouth is set at such a length that the admission port of the mouthpiece may be placed in the range from the vicinity of the gustatory organs which sense bitterness to the deep part of the oral cavity. This enables the medicine powder to be discharged in the position beyond the deep part of the tongue which senses bitterness. For this reason, the medicine powder can be inhaled comfortably while the bitterness of the medicine powder is not being sensed.

### BRIEF DESCRIPTION OF THE DRAWINGS

Fig. 1 is a longitudinal sectional view showing an inhalation-type medicine applicator, to which a long mouthpiece is attached, according to the present invention.
Fig. 2 is a right side view of the medicine applicator main body from which a capsule holder is detached.
Fig. 3 is a magnified, longitudinal sectional view showing the capsule holder in a magnified manner.
Fig. 4 is a transverse cross-section of the capsule holder shown from the arrowed IV-IV direction of Fig. 3.
Fig. 5 is a longitudinal sectional view showing the inhalation-type medicine applicator in a condition where the air is inhaled through the long mouthpiece.
Fig. 6 is a magnified, cross-sectional view showing a primary part of the internal anatomy of the mouth in which the mouthpiece is held.
Fig. 7 is an outside view of the tongue showing a position of the sweetness sensing parts.
Fig. 8 is the outside view of the tongue showing a position of the saltiness sensing parts.
Fig. 9 is the outside view of the tongue showing a position of the sourness sensing parts.
Fig. 10 is the outside view of the tongue showing a position of the bitterness sensing parts.

### DETAILED DESCRIPTION OF THE PREFERRED EMBODIMENTS

Next, descriptions will be provided for an inhalation-type medicine applicator according to an embodiment of the present invention with reference to the drawings.

It should be noted that, the gist of the present embodiment is that an admission port of a mouthpiece can be set in a way that the admission port may be placed in a range out of areas on the human tongue where gustatory organs to sense sweetness, sourness and bitterness are present, and that accordingly medicine powder can be discharged from the admission port of the mouthpiece in a position out of the areas on the tongue which sense sweetness, sourness and bitterness.

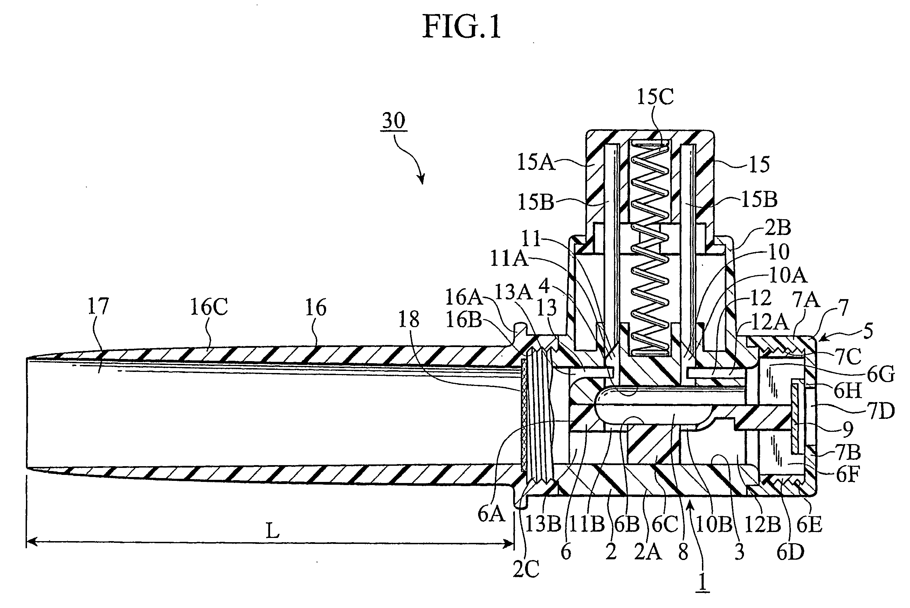

Hereinafter, detailed descriptions will be provided for the inhalation-type medicine applicator according to the present invention, citing a case where medicine powder is administered to a patient suffering, for example, from bronchial asthma, with reference to Figs. 1 to 10.

As shown in Fig. 1, the inhalation-type medicine applicator 30 according to the present embodiment is chiefly configured to include a medicine applicator main body 1 which constitutes a base for this inhalation-type medicine applicator 30, and a mouthpiece 16 which is attached to this medicine applicator main body 1. The medicine applicator main body 1 includes a body 2 shaped like a cylinder, a capsule holder 5, a capsule container 9 and the like.

The body 2 is constituted of a cylinder-shaped body 2A which is formed virtually in a cylinder, a hole-maker guide 2B which is installed to this cylinder-shaped body 2A, and which protrudes from the outer periphery of the cylinder-shaped body 2A. This hole-maker guide 2B supports a supporting part 15A, which will be described later, of a hole maker 15 shaped like a cylinder, in a way that the supporting part 15A can slide (come out and in) in a direction orthogonal to the axis direction of the cylinder-shaped body 2A. A screw part 2C to which the mouthpiece 16 is screwed is provided, with a screw thread of the screw part 2C formed, in the outer periphery of an end part in the axis direction of the cylinder-shaped body 2A. The inside of the cylinder-shaped body 2A constitutes a holder containing part 3.

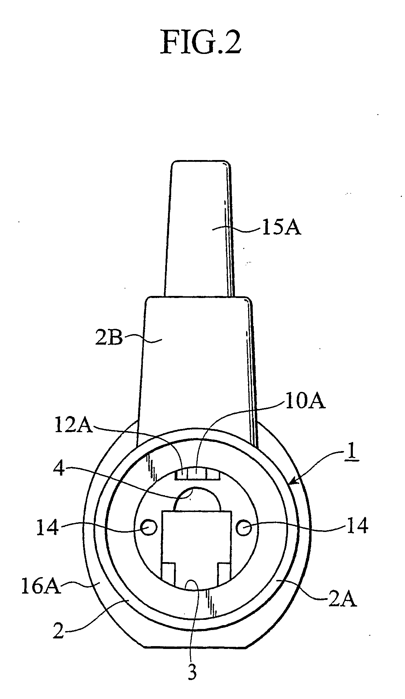

This holder containing part 3 contains a capsule holding part 6 of the capsule holder 5 in a way that the capsule holding part 6 can come out of, and into, the holder containing part 3. In addition, the holder containing part 3 is positioned on the lower side of the cylinder-shaped body 2A, as shown in Fig. 2. The holder containing part 3 is formed as a through-hole which extends in the axis direction of the cylinder-shaped body 2A. Furthermore, the cross-section of this holder containing part 3, which is orthogonal to the axis direction of the cylinder-shaped body 2A is shaped, virtually, like a letter of T as shown in Fig. 2.

The capsule fitting groove 4 is formed in the upper surface of the inside of this holder containing part 3, as shown in Figs. 1 and 2. Along with a capsule fitting concave part 6B of the capsule holding part 6 which will be described later, this capsule fitting groove 4 constitutes the capsule container 8 as a medicine powder container. In addition, the capsule fitting groove 4 holds a capsule K from the above with the inner side in the groove thereof, and is formed as a groove whose cross section is shaped like a half arc corresponding to the external measurement of the capsule K.

The capsule holder 5 is detachably provided to the body 2. In addition, the capsule holder 5, along with the body 2 and the like, constitutes the medicine applicator main body 1. Furthermore, the capsule holder 5 is constituted chiefly of the capsule holding part 6, a cap 7 and the check valve 9.

The capsule holding part 6 is a part of the capsule holder 5, and is provided to the holder containing part 3 in a way that the capsule holding part 6 can come out of, and into, the holder containing part 3. Specifically, as shown in Figs. 1, 3 and 4 , the capsule holding part 6 is constituted chiefly of a drawing part 6A formed to extend in the axis direction in the upper portion in the holder containing part 3; the capsule fitting concave part 6B formed as a groove, whose cross section is shaped like a half arc, and which is long in the axis direction, in the upper surface of the drawing part 6A; a guiding part 6C protruding downward from the drawing part 6A; an annular part 6D formed in a circle to surround one end of the drawing part 6A; the screw part 6E whose screw thread is formed in the outer periphery of this annular part 6D; a plurality (four in the present embodiment) of legs 6F formed in a radial manner in order to connect the annular part 6D to the drawing part 6A; openings 6G respectively constituting air paths formed between each neighboring two of the legs 6F; and a depression part 6H formed by notching a center portion of each of the legs 6F towards one side.

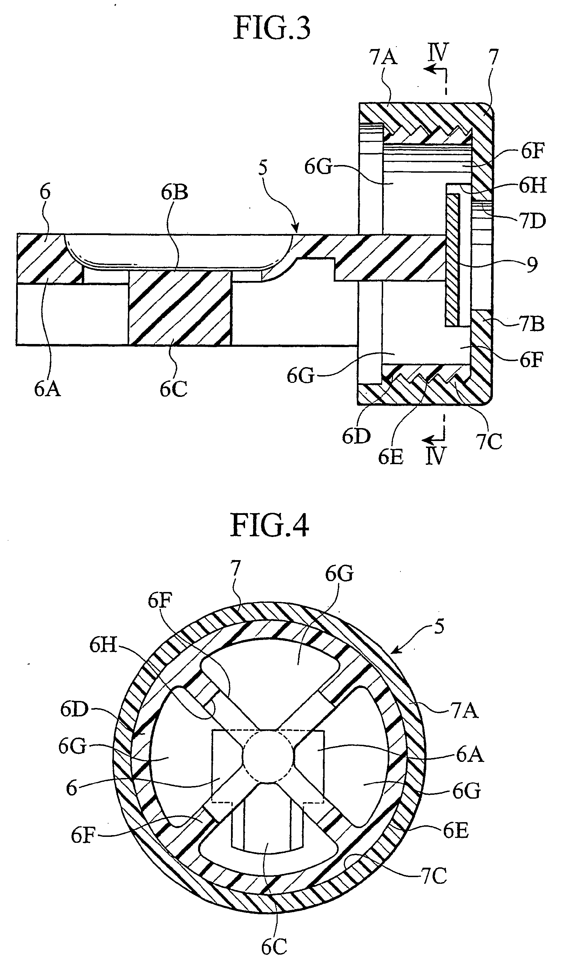

The cap 7 is attached to the capsule holding part 6 in a way that the cap 7 covers the annular part 6D and the like of the capsule holding part 6. As shown in Figs. 1 and 3, the cap 7 is formed of a cylinder part 7A and a lid part 7B, and is shaped like a cylinder with a lid. In the internal periphery of the cylinder part 7, a screw part 7C which is screwed into the screw part 6E of the capsule holding part 6E is formed. Furthermore, the center of the lid part 7B is provided, for example, with an air intake 7D shaped like a circle. This air intake 7 takes in the air from the outside, and supplies the air to each of the openings 6G of the capsule holding part 6 and to an inflow-side path 12

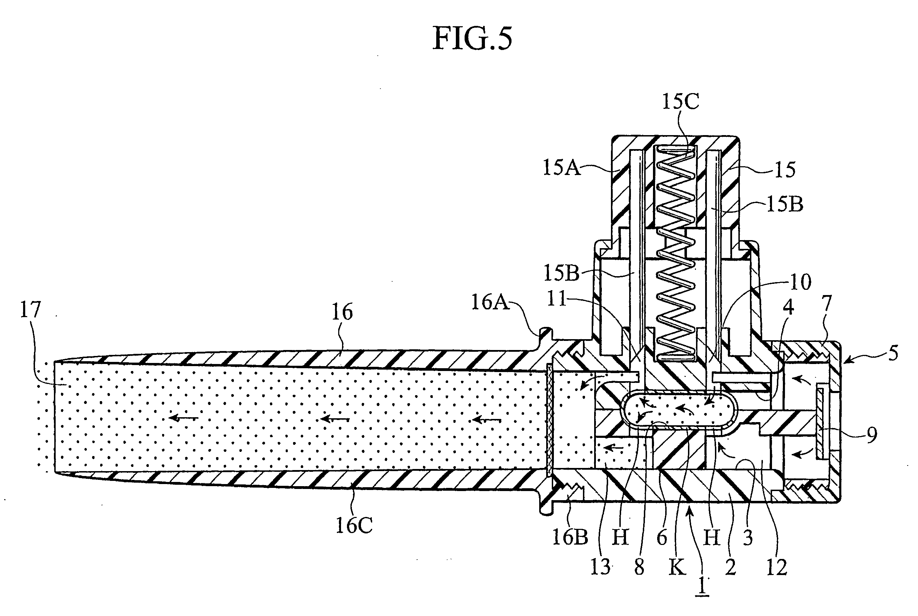

As shown in Figs. 1 and 5, the capsule container 8 is a medicine powder container which is formed between the capsule fitting groove 4 and the capsule fitting concave part 6B when the capsule holding part 6 is pressed into the holder containing part 3. This capsule container 8 contains, and holds, the capsule K, as shown in Fig. 5.

The check valve 9 is provided between the capsule holding part 6 and the cap 7. The check valve 9 is a disc body arranged in the depression part 6H. In addition, the check valve 9 closes the air intake 7D when a user of the inhalation-type medicine applicator has a fit of coughing while inhaling the medicine powder, thereby preventing the medicine powder from flowing back due to the user's fit of coughing.

The capsule holder 5 thus formed can arrange the capsule holding part 6 in a drawing position, if the capsule holding part 6 is drawn out while the outer periphery of the cap 7 is being held. In addition, in this drawing position, a capsule K which has been filled with medicine powder can be fitted into the capsule fitting concave part 6B formed in the drawing part 6A, and a capsule K which has been used can be removed from the capsule fitting concave part 6B.

Furthermore, if the drawing part 6A is pressed into the holder containing part 3 while the capsule K is being fitted to the capsule fitting concave part 6B, the capsule holding part 6 is arranged in the pressed position, and thereby the capsule K can be held in the capsule container 8, as shown in Fig. 5.

As shown in Fig. 1, the capsule container 8 is provided with a pin inserting hole 10 on the inflow side formed in a position towards one side, and with a pin inserting hole 11 on the outflow side formed in a position towards the other side. The pin inserting hole 10 is constituted of: a body-side insertion hole 10A formed in the body 2 in a way that the body-side insertion hole 10 penetrates the capsule container 8 in the diameter direction thereof; and a holder-side insertion hole 10B formed in the capsule holder 5. In addition, the pin inserting hole 11 is constituted of: a body-side insertion hole 11A which penetrates the capsule container 8 in parallel with the pin inserting hole 10 on the inflow side; and a holder-side insertion holellB.

An inflow-side path 12 through which the air is flown from the capsule holder 5 is formed in the capsule holding part 6 in a way that the inflow-side path 12 connects with the capsule holder 8. This inflow-side path 12 is constituted of: a body-side path 12A connecting with the pin inserting hole 10 on the inflow side; and a holder-side path 12B which is provided between the holder containing part 3 and the capsule holding part 6 separately from the body-side path 12A. In addition, the inflow-side path 12 is configured to be capable of connecting with the atmosphere through each of the openings 6G of the capsule holding part 6 and the air intake 7D of the cap 7.

Moreover, an outflow-side path 13 through which the air containing medicine powder is flown out to the admission port 17, which will be described later, is configured to connect with the capsule container 8. This outflow-side path 13 is constituted of a body-side path 13A connecting with the pin inserting hole 11 on the inflow side, and a holder-side path 13B which is provided between the holder containing part 3 and the capsule holding part 6 separately from the body-side path 13A.

As shown in Fig. 2, two auxiliary air paths 14 are formed to penetrate the cylinder-shaped body 2A of the body 2 in the axis direction thereof in the respective positions shifted respectively from the paths 12 and 13 by 90°. Each of the auxiliary air paths 14 relieves a choking sensation, which the user of this inhalation-type medicine applicator feels, by increasing an amount of the air flowing when the user inhales his/her breath.

The hole maker 15 is provided to the hole-maker guide 2B, which is formed in the body 2 as described above. This hole maker 15 makes holes in the capsule K contained in the capsule container 8. In addition, as shown in Fig. 1, the hole maker 15 is constituted chiefly of: a supporting part 15A supported movably in the hole-maker guide 2B of the body2; pins 15B and 15B extending to the respective pin inserting holes 10 and 11 from the supporting part 15A; and a return spring 15C provided between the supporting 15A and the cylinder-shaped body 2A. Incidentally, the base of the hole-maker 15 is formed to have a structure which does not allow the base of the hole maker 15 to exit from the hole-maker guide 2B.

In addition, the return spring 15C applies a force to the supporting part 15A in the same direction as each of the pins 15B come apart from the capsule K. The force thus applied causes the supporting part 15A and each of the pins 15B to return to their respective initial positions after the holes are made in the capsule K. Furthermore, the tip of each of the pins 15 is shaped like a sharp needle tip having an inclined plane.

As shown in Fig. 5, the hole maker 15 presses the supporting part 15A into the hole-maker guide 2B against the return spring 15C, and inserts the pins 15B and 15B respectively into the pin inserting holes 10 and 11. Thereby, as shown in Fig. 5, the hole maker 15 pierces the tips respectively of the pins 15B into the capsule K in the capsule container 8, thus making four holes H which penetrate the capsule K in the diameter direction of thereof.

The mouthpiece 16 is formed as a long cylinder body which extends in the axis direction. The inside of the mouthpiece 16 is an admission port 17 through which medicine powder is inhaled. In addition, the other end part of the mouthpiece 16 is provided with a flange part 16A. A screw part 16B which is screwed into screw part 2C of the body 2 is provided, with a screw thread of the screw part 16B formed, in the internal periphery on the side opposite to this flange part 16A. Furthermore, a part of the mouthpiece 16 extending towards one end from the flange part 16A is a held-in-the-mouth area 16C which is held in the mouth. The diameter of the held-in-the-mouth area 16C becomes gradually smaller towards the other end thereof. Moreover, a mesh member 18 for diffusing medicine powder and for catching broken pieces of the capsule and the like is provided to a deeper inside of the mouthpiece 16.

### [Descriptions of Areas in the Tongue Which Sense Taste]

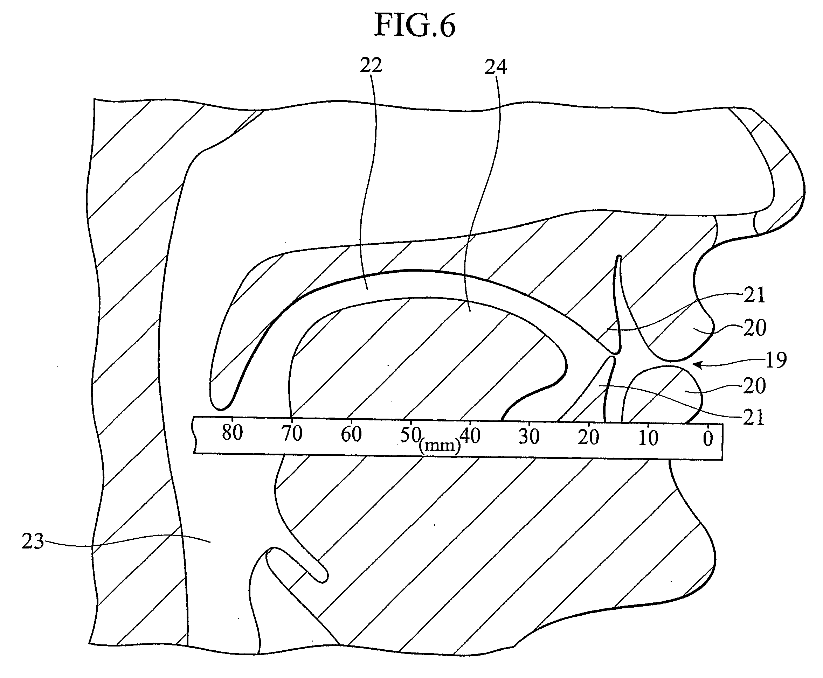

Herein below, descriptions will be provided for the anatomy of the human mouth 19 into which the held-in-the-mouth area 16C is inserted with reference to Figs. 6 to 10, before descriptions are provided for a length L of the held-in-the-mouth area 16C constituting the mouthpiece 16.

As shown in Fig. 6, the mouth 19 has the upper lip 20 and the lower lip 20 outside the mouth 19. Upper and lower front teeth 21 are arranged in positions inwards respectively from the lips 20 by approximately 20mm. In addition, the inside beyond the front teeth 21 expands as the oral cavity 22. The deepest part of the oral cavity 22 (a position approximately 80mm away from the lips 20) is the pharynx 23 leading to the esophagus and the bronchi. Furthermore, the tongue 24 exists in the base in the oral cavity 22. The tongue 24 includes various areas having gustatory organs which sense taste of matters which are put into the oral cavity 22.

### (Sweetness Sensing Areas)

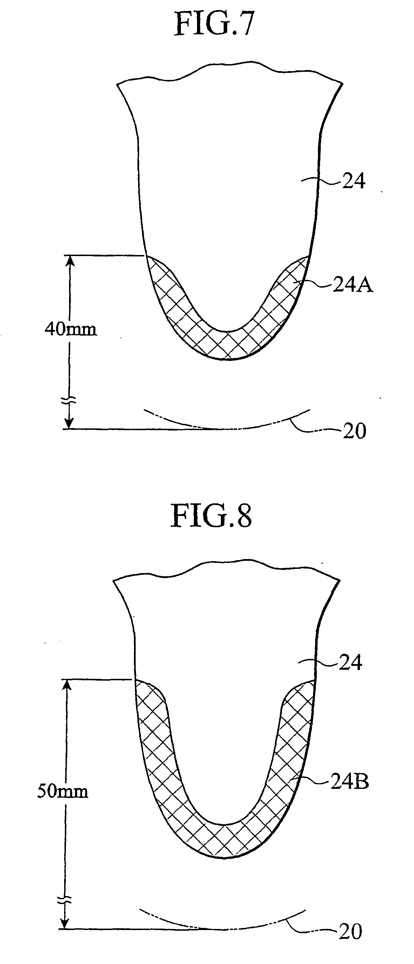

Sweetness sensing parts 24A which sense sweetness exist in the front ends of the tongue 24, as shown in Fig. 7. The sweetness sensing parts 24A are arranged up to a position inside approximately 40mm from the lips 20.

### (Saltiness Sensing Areas)

Saltiness sensing parts 24B which sense saltiness exist in a position from the front ends to the right and the left edges of the tongue 24, as shown in Fig. 8. This saltiness sensing parts 24B are arranged up to a position inside approximately 50mm from the lips 20.

### (Sourness Sensing Areas)

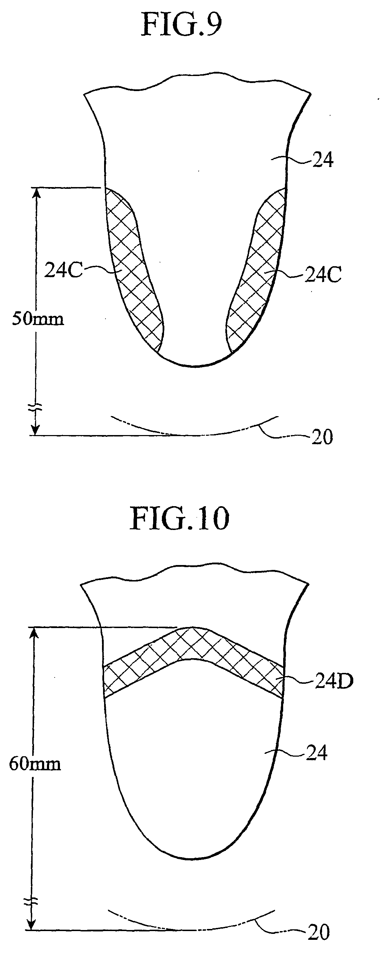

Sourness sensing parts 24C which sense sourness exist in the right and the left side edges of the tongue 24, as shown in Fig. 9. This sourness sensing parts 24C are arranged up to a position inside approximately 50mm from the lips 20.

### (Bitterness Sensing Areas)

Bitterness sensing parts 24D which sense bitterness exist in the deep parts of the tongue 24 in a way that the bitterness sensing parts 24D go across the tongue 24, as shown in Fig. 10. This bitterness sensing parts 24A are arranged up to a position inside approximately 60mm from the lips 20.

Each group of the sensing parts 24A to 24D constituting gustatory organs has an important role of sensing taste of food and a drink when the food and the drink put into the oral cavity 22 adhere to each group of the sensing parts 24A and 24D. However, each of the group of the sensing parts 24A and 24D senses taste of medicine powder, which needs not be sensed, when the medicine powder adheres to each of the group of the sensing parts 24A and 24D. Accordingly, a patient may feel discomfort while inhaling the medicine powder.

With this taken into consideration, the length L in the axis direction of the held-in-the-mouth area 16C of the mouthpiece 16 according to the present embodiment is set at approximately 80mm. If the length L of the held-in-the-mouth area 16C were set at approximately 80mm in this manner, when the held-in-the-mouth part 16C is inserted deep into the oral cavity 22 until the flange part 16A comes to abut to the lips 20, the end part of the held-in-the-mouth part 16C can be open to a position beyond the bitterness sensing parts 24D which are located the deepest in the tongue. As a result, even though medicine powder to be discharged from the admission port 17 includes sweetness, saltiness, sourness and bitterness, the medicine powder can be administered to an affected part in the lungs or the like through the pharynx 23 while the taste of the medicine powder is not being sensed.

In addition, if the length L of the held-in-the-mouth area 16C were set at the longer 80mm, the held-in-the-mouth area 16C can press down the tongue 24 which is an obstacle in the oral cavity 22. This enables the medicine powder to be discharged directly to the pharynx 23.

On the other hand, if the long mouthpiece 16 were detached, and if a mouthpiece (not illustrated) of a middle length whose held-in-the-mouth is measured approximately 50mm in the axis direction thereof were attached instead, the end part of the held-in-the-mouth area 16C can be open to a position beyond the sweetness sensing parts 24A., the saltiness sensing parts 24B and the sourness sensing parts 24C. In this manner, the mouthpiece of the middle length is suitable for inhaling medicine power which is sweetness, saltiness or sourness, but not bitterness.

Furthermore, in a case where a mouthpiece of a short length which is shorter than the mouthpiece of the middle length is attached, if the length of the held-in-the-mouth area in the axis direction were set at approximately 40mm, the medicine powder can be inhaled while sweetness stemming from sweet ingredients is not being sensed.

Next, descriptions will be provided for a motion in which a patient inhales medicine powder in a case where the long mouthpiece 16 is used for the inhalation-type medicine applicator 30 according to the present embodiment.

First of all, the capsule holder 5 is drawn out of the holder containing part 3, and the capsule K is fitted to the capsule fitting concave part 6B of the capsule holding part 6. While this state is being kept, the capsule holding part 6 is pressed into the holder containing part 3, and the capsule K is set in the capsule container 8. Subsequently, the supporting part 15A of the hole-maker 15 is pressed in along the hole-maker guide 2B. Thereby, the pins 15B make the respective four holes H in the capsule K.

After each of the holes is made in the capsule K in the capsule container 8 in this manner, the patient holds the held-in-the-mouth area 16C of the long mouthpiece 16 in his mouth, and inhales a breath. Thereby, the medicine powder can be administered to the bronchi respectively of the lungs and the like through the pharynx 23. In this occasion, since the length L of the held-in-the-mouth area 16C in the axis direction thereof is set at 80mm, the patient can inhale the medicine powder discharged from the admission port without sensing the taste of the medicine powder due to sweet, salty, sour and bitter ingredients.

As described above, according to the present embodiment, with regard to the long mouthpiece 16 which is held in the mouth 19 when the medicine powder is inhaled, the length of the held-in-the-mouth area 16C which is held in the mouth is set at approximately 80mm. For this reason, when the held-in-the-mouth area 16C is held in the mouth, its end part can be inserted deep into the oral cavity 22. Accordingly, the medicine powder can be discharged in a position beyond the sweetness sensing parts 24A, the saltiness sensing parts 24B, the sourness sensing parts 24C and the bitterness sensing parts 24D which are provided to the tongue 24. As a consequence, the patient can inhale the medicine powder comfortably without sensing the taste of the medicine powder such as bitterness, sourness and sweetness while the patient is inhaling the medicine powder.

Moreover, if the held-in-the-mouth area 16C of the long mouthpiece 16 were formed to have a larger length, it can press down the tongue 24 which is an obstacle, and accordingly can discharge the medicine powder directly to the pharynx 23. Thereby, the medicine powder can be prevented from adhering to the tongue 24, the internal surface of the oral cavity 22 and the like. Thus, a prescribed amount of medicine powder can be administered to the bronchi respectively of the lungs and the like efficiently.

### (Other Embodiments)

The descriptions and the drawings which constitute parts of the disclosure of the aforementioned embodiment should not be interpreted as limiting the present invention. Various alternative embodiments, examples and applied techniques may be apparent from this disclosure to those skilled in the art.

The aforementioned embodiment has been described giving the case, for example, of the administering of medicine powder to a patient suffering from bronchial asthma by use of the inhalation-type medicine applicator. However, it goes without saying that the inhalation-type medicine applicator can be applied to the administering of medicine powder in other cases.

In addition, the aforementioned embodiment has been described giving an example where the length L of the held-in-the-mouth area 16C of the mouthpiece 16 of a larger length is set at approximately 80mm, the length of the held-in-the-mouth area of the mouthpiece of the middle length is set at approximately 50mm, and the length of the held-in-the-mouth area of the mouthpiece of a shorter length is set at approximately 40mm. However, the length of the held-in-the-mouth area is not limited to these. For example, lengths of the held-in-the-mouth area can be set in a wider range including a range of 30mm to 80mm whenever necessary for each purpose depending on sweet, salty, sour, bitter and other ingredients included in medicine powder.

Moreover, according to the aforementioned embodiments, the inhalation-type medicine applicator is configured to contain a capsule K, which is filled with medicine powder, in the capsule container 8. However, the present invention is not limited to this embodiment. For example, the inhalation-type medicine applicator may have a configuration in which the medicine applicator main body is provided with a medicine powder container, accordingly the medicine powder container is filled directly with medicine powder, and thus the medicine powder is inhaled.

### INDUSTRIAL APPLICABILITY

The present invention enables the end of the mouthpiece to be inserted deep into the oral cavity when the mouthpiece is held in the mouth. This enables the medicine powder to be discharged, for example, in a position beyond the gustatory organs. Thereby, the medicine powder can be inhaled comfortably while taste of the medicine powder including bitterness, sourness, sweetness and the like is not being sensed when the medicine powder is inhaled.

In addition, the mouthpiece can press down the tongue which is an obstacle, thereby enabling the medicine powder to be discharged directly to the respiratory tract. This can prevent the medicine powder from adhering to the tongue, the interior of the oral cavity and the like. Accordingly, a prescribed amount of medicine powder can be administered to an affected part in the bronchi or the like efficiently.

The length of the part of the mouthpiece which is held in the mouth is set in the range of 40mm to 80mm in a case where medicine powder to be inhaled is medicine powder including sweetness. This enables the mouthpiece to discharge the medicine powder in a position beyond the front ends of the tongue which senses sweetness. For this reason, the medicine powder can be inhaled comfortably while the sweetness of the medicine powder is not being sensed.

Furthermore, the length of the part of the mouthpiece which is held in the mouth is set at the range of 50mm to 80mm in a case where medicine powder to be inhaled is medicine powder including sourness. This enables the mouthpiece to discharge the medicine powder in a position beyond the side edges of the tongue which senses sourness. For this reason, the medicine powder can be inhaled comfortably while the sourness of the medicine powder is not being sensed.

Moreover, the length of the part of the mouthpiece which is held in the mouth is set at the range of 60mm to 80mm in a case where medicine powder to be inhaled is medicine powder including bitterness. This enables the mouthpiece to discharge the medicine powder in a position beyond the deep part of the tongue which senses bitterness. For this reason, the medical powder can be inhaled comfortably while the bitterness of the medicine powder is not being sensed. The length of the part of the mouthpiece which is held in the mouth is set at such a length that the admission port of the mouthpiece may be placed in the range from the vicinity of the gustatory organs, which sense sweetness, to the deep part of the oral cavity. This enables the medicine powder to be discharged in a position beyond the front ends of the tongue which sense sweetness. For this reason, the medicine powder can be inhaled comfortably while the sweetness of the medicine powder is not being sensed.

Moreover, the length of the part of the mouthpiece which is held in the mouth is set at such a length that the admission port of the mouthpiece may be placed in the range from the vicinity of the gustatory organs which sense sourness to the deep part of the oral cavity. This enables the medicine powder to be discharged in a position beyond the side edges of the tongue which sense sourness. For this reason, the medicine powder can be inhaled comfortably while the sourness of the medicine powder is not being sensed.

Additionally, the length of the part of the mouthpiece which is held in the mouth is set at such a length that the admission port of the mouthpiece may be placed in the range from the vicinity of the gustatory organs which sense bitterness to the deep part of the oral cavity. This enables the medicine powder to be discharged in a position beyond the deep part of the tongue which sense bitterness. For this reason, the medicine powder can be inhaled comfortably while the bitterness of the medicine powder is not being sensed.

## Claims

1. An inhalation-type medicine applicator comprising:
a medicine powder container configured to contain medicine powder therein; and
a mouthpiece configured to be held in the mouth when the medicine powder in the medicine powder container is inhaled,
wherein
the mouthpiece includes a part held in the mouth, the part is set in a rage of 30mm to 80mm in length.

2. The inhalation-type medicine applicator according to claim 1,
wherein
the part held in the mouth is set in a rage of 40mm to 80mm in length in a case where medicine powder to be inhaled is medicine powder including sweetness.

3. The inhalation-type medicine applicator according to claim 1, wherein
the part held in the mouth is set in a rage of 50mm to 80mm in length in a case where medicine powder to be inhaled is medicine powder including sourness.

4. The inhalation-type medicine applicator according to claim 1,
wherein
the part held in the mouth is set in a rage of 60mm to 80mm in length in a case where medicine powder to be inhaled is medicine powder including bitterness.

5. An inhalation-type medicine applicator comprising:
a medicine powder container configured to contain medicine
powder therein; and
a mouthpiece configured to be held in the mouth when the
medicine powder in the medicine powder container is inhaled,
wherein
the mouthpiece includes an admission port which is set to be placed in a range from the vicinity of gustatory organs sensing sweetness to a deep part of the oral cavity when the mouthpiece is held in the mouth.

6. An inhalation-type medicine applicator comprising:
a medicine powder container configured to contain medicine
powder therein; and
a mouthpiece configured to be held in the mouth when the
medicine powder in the medicine powder container is inhaled,
wherein
the mouthpiece includes an admission port which is set to be placed in a range from the vicinity of gustatory organs sensing sourness to a deep part of the oral cavity when the mouthpiece is held in the mouth.

7. An inhalation-type medicine applicator comprising:
a medicine powder container configured to contain medicine powder therein; and
a mouthpiece configured to be held in the mouth when the medicine powder in the medicine powder container is inhaled,
wherein
the mouthpiece includes an admission port which is set to be placed in a range from the vicinity of gustatory organs sensing bitterness to a deep part of the oral cavity when the mouthpiece is held in the mouth.

8. An inhalation-type medicine applicator comprising:
a body;
a medicine powder container configured to contain medicine powder in the body; and
a mouthpiece configured to be held in the mouth when the
medicine powder in the medicine powder container is inhaled,
wherein
the mouthpiece with a different length from the body is configured to be attachable to, and detachable from, the body.
